# EUROPEAN PATENT APPLICATION

(11) **EP 2 712 536 A1**
(43) Date of publication of application: **02.04.2014**
(21) Application number: 13186234.4
(22) Date of filing: 26.09.2013
(51) Int. Cl.: A61B 1/00, A61B 1/12

(54) **Telescope antifogging and defogging system**

(30) Priority: 27.09.2012 US 201261706432 P
(71) Applicant: Karl Storz Endoscopy-America, Inc., El Segundo, CA 90245 (US)
(72) Inventor: Solingen, Simon, El Segundo, 90245 (US)
(74) Representative: Witte, Weller & Partner Patentanwälte mbB

(57) **Abstract**

A system for preventing or removing fogging from and endoscope which includes an endoscope having a distal end and a heating module disposed over the distal end of the endoscope. The heating module may include a heating element arranged as a single-segment heater, or in a series of elongated portions extending circumferentially around the distal end of the endoscope connected by bend portions that are parallel or tangent to a longitudinal axis of the endoscope. The heating module may also be disposed on or adhered to an outside surface of the endoscope, and may include a heating element made from a resistive heating material. The heating module may be provided as a single-use disposable device, and may include an outside surface made from a biocompatible or sterilizable insulating material.

## Description

### FIELD OF THE INVENTION

The invention relates to endoscopes in general, and more particularly, to preventing or removing fogging on a distal optical lens or window of an endoscope.

### BACKGROUND OF THE INVENTION

Fogging is a term commonly used to describe the condensation of liquid water droplets on a clear surface, which can scatter light and obscure the transparency of the surface. Fogging of smooth clear surfaces is a common occurrence, and can cause problems in applications where clarity is important, such as in devices which incorporate optical lenses.

Medical instruments incorporating windows or lenses and which are employed within body cavities, such as endoscopes, are particularly prone to fogging. Changes in temperature and pressure which can be caused by the relatively warm and humid environment of a body cavity or by the introduction of insufflation gas at a particular temperature, pressure, and humidity can promote the formation of condensation on the surfaces of a relatively cool surface, such as the objective lens or window of an endoscope.

For these reasons, fogging is a common nuisance during endoscopic surgery. Unexpected fogging of an optical device under these circumstances may result in surgical errors or delays in completing the surgery which can complicate or prolong recovery.

Many approaches to preventing or removing optical element fogging are known, and include the application of anti-fogging agents, preheating the lens or optical window before insertion, and dehumidification, for example, by supplying warmed insufflation gas flowing over the lens or optical window.

However, applied anti-fogging agents are prone to gradual displacement by liquids or abrasion, reducing durability and dependability. Warmed insufflation gas flowing over the lens or optical window requires additional hardware to supply, increasing cost and complexity. Furthermore, in laparoscopy or thoracoscopy, adding hardware to supply warmed insufflation gas to the lens or optical window requires a trocar-cannula of a larger diameter to accommodate the additional hardware. This requires a larger incision, which is undesirable in these operations. Hitherto known warming systems are also deficient in that they do not transfer heat in an advantageous manner. Warming systems that are incorporated inside endoscopes require substantial changes to the device's design and do not address the needs of an installed base of tens of thousands of endoscopes.

What is desired therefore is a system which addresses these deficiencies.

### SUMMARY OF THE INVENTION

Accordingly, it is an object of the present invention to provide a defogging system which prevents fogging of lenses or windows disposed in a distal end of an endoscope.

It is a further object of the present invention to provide an anti-fogging system which removes fogging of lenses or windows disposed in a distal end of an endoscope.

These and other objectives are achieved by providing a system which includes an endoscope having a distal end and a heating module disposed external to, and circumferentially around or longitudinally coaxial with, the distal end of the endoscope.

In some embodiments, the heating module includes a heating element arranged in a series of elongated portions extending circumferentially, covering an arc around the distal end of the endoscope connected by bend portions that are parallel or tangent to a longitudinal axis of the endoscope.

In some embodiments, the heating module includes a heating element having one segment extending circumferentially over an arc around the distal end of the endoscope.

In some embodiments, the heating module includes a heating element having more than one segment extending circumferentially over an arc around the distal end of the endoscope.

In some embodiments, the heating module is adhered to an outside surface of the endoscope.

In some embodiments, the heating module is disposed on an outside surface of the endoscope.

In some embodiments, the heating module includes a heating element made from a resistive or other heating material.

In some embodiments, the heating module includes an outside surface made from a biocompatible insulating material.

In some embodiments, the heating module includes a heating element made using thin film technology, or thick film semi-conductive ink.

In some embodiments, the electrical conductuctors are made using thin film technology, or thick film conductive ink.

In some embodiments, the electrical conductors or heating element are made using a silk screen printing process.

In some embodiments, the electrical conductors or heating element are made using a chemical or electrochemical etching process.

In some embodiments, the heating element is made from a material having an electrical resistance with a positive temperature coefficient to form a self-limiting heating element.

Other objects are achieved by providing a method for preventing or removing fogging from an endoscope by providing a heating module and attaching the heating module to an outside surface of a distal end of an endoscope.

Further objects are achieved by providing a system for preventing or removing fogging from an endoscope which includes an endoscope having a distal end; and, a heating module disposed over the distal end of the endoscope on an outside surface of the endoscope, which includes a heating element that is positioned between two insulating ribbons and made from a resistive heating material which is self-limiting such that its temperature will not exceed a certain maximum.

These and other objects of the invention and its particular features and advantages will become more apparent from consideration of the following drawings and accompanying detailed description.

### BRIEF DESCRIPTION OF THE DRAWINGS

FIG. 1 illustrates an endoscope heating device according to aspects of the invention.

FIG. 2 illustrates the endoscope heating device shown in FIG. 1 in an example installation on an endoscope according to aspects of the invention.

FIG. 3 is an enlarged view of a portion of the endoscope heating device illustrated in FIG. 1 and FIG. 2.

FIG. 4 illustrates the endoscope heating device shown in FIGS. 1 - 3 including an additional component according to aspects of the invention.

FIG. 5 is an enlarged view of a portion of the endoscope heating device illustrated in FIGS. 1 - 4.

FIG. 6 is a cross-sectional view of a portion of the endoscope heating device illustrated in FIGS. 1 - 5.

FIGS. 7 - 11 are enlarged views which illustrate alternative configurations for the endoscope heating device shown in FIGS. 1 - 6.

### DETAILED DESCRIPTION OF THE INVENTION

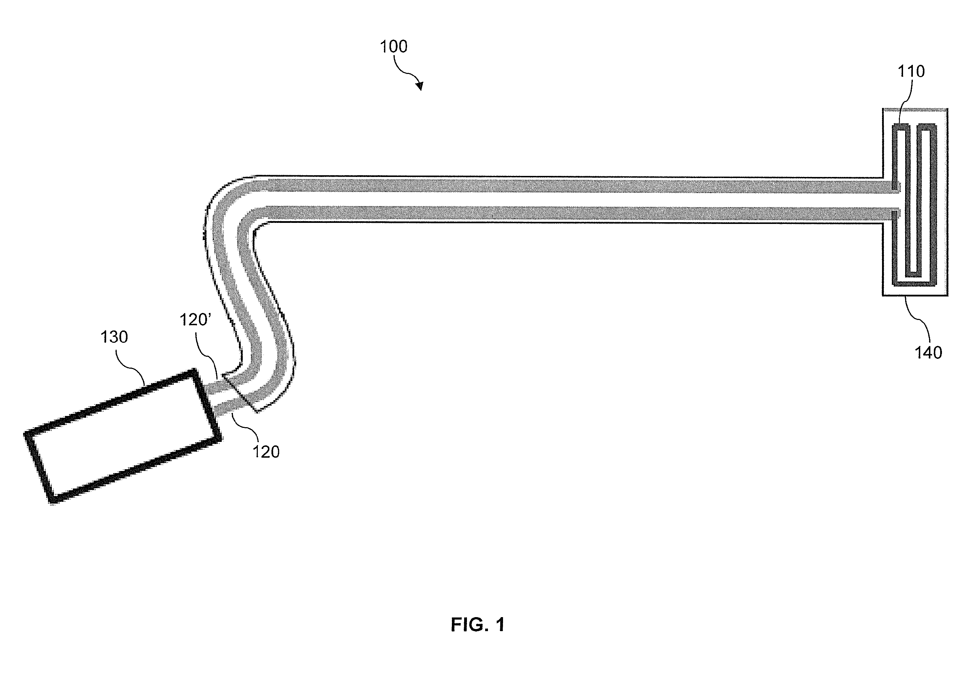

FIG. 1 illustrates an example endoscope defogging and anti-fogging device 100 according to aspects of the invention.

Device 100 includes a heating element 110, electrical conductors 120, 120', power source 130, and insulation 140.

Heating element 110 may be a wire coil, trace, film, or other suitable structure and may be made from a material which heats when a sufficient current is applied. Suitable materials include Nichrome, Constantan, Carbon, Iron, semiconducting ink, or any other material or alloy commonly used in constructing resistors.

Electrical conductors 120 and 120' are wires or traces made from a conducting material suitable for delivering power to resistive heating element 110 while maintaining a reasonably low level of resistive heating and voltage drop within electrical conductors 120 and 120'

Power source 130 supplies power to the heating element 110 via conductors 120, 120'. Heating element 110 may be self-limiting such that regardless of the temperature of the environment surrounding heating element 110, the power supplied to heating element 110 from power source 130 will be adjusted to prevent the temperature of heating element 110 from exceeding a certain maximum. Optionally, the power delivered to heating element 110, and the heat supplied by heating element 110, may be controlled by a manual adjustment mechanism, computer controlled mechanism, or a negative feedback servomechanism control, for example. Other mechanisms for controlling power delivery and temperature will be evident to those having skill in the art.

In some applications one or more, or all components of device 100 may be provided as a single-use disposable device. In some applications, heating element 110 and electrical conductors 120, 120' may be provided as a single-use disposable device. In some applications, heating element 110, electrical conductors 120, 120' and power source 130 may be provided as a single-use disposable device.

Insulation 140 may be made from a suitable insulator such as Polyimide, Fluoropolymer (FEP, PFA, ETFE), PVC, TPE, Polyurethane (TPU), Silicone Rubber, Polyethylene, Polypropylene, Polyester, Nylon, or other insulator. In applications where insulation 140 will come into contact with human or animal body tissues or cavities, the insulator may be chosen such that it is biocompatible and/or sterilizable. In some applications, portions of insulation 140 may be formed as a flat ribbon encasing electrical conductors 120, 120' and/or resistive heating element 110. In some applications, portions of insulation 140 may include two or more ribbons enclosing heating element 110 and or electrical conductors 120, 120'. In some applications, insulation 140 may conform to the shape of the electrical conductors, such as when electrical conductors 120, 120' and insulation 140 are not required to run along the outside surface of a medical device, or where a recess is provided to run these components, for example. Other configurations of insulation 140 will be evident to those having skill in the art.

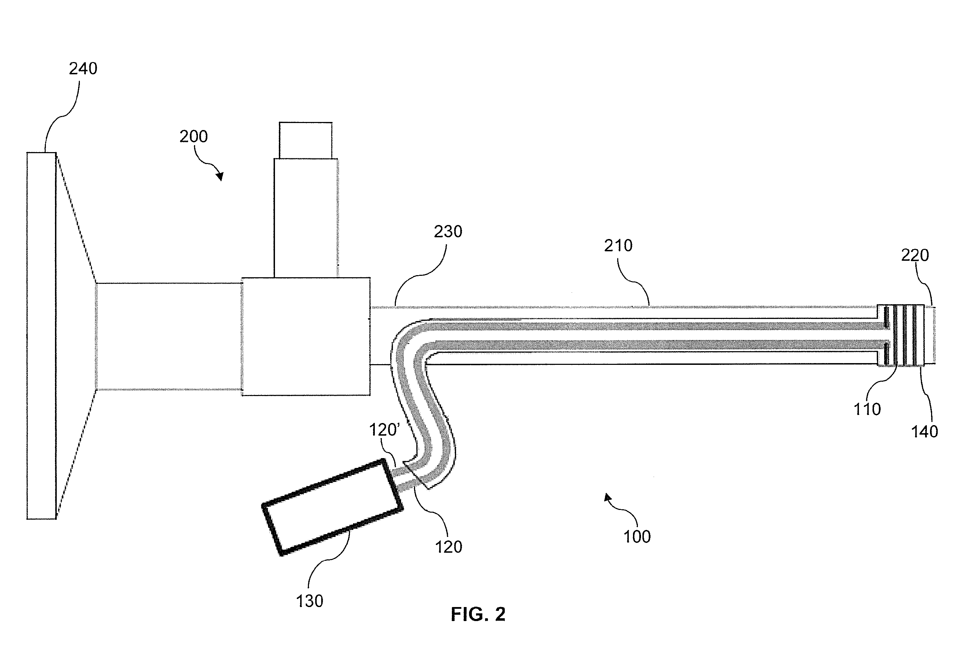

FIG. 2 illustrates an example system according to aspects of the invention which includes an installation of endoscope heating device 100 on an example endoscope 200.

Endoscope 200 includes a shaft 210, distal end 220, proximal end 230, and an eyepiece 240. Distal end 220 includes an objective lens, window, or other optic or smooth transparent surface that is subject to fogging (not shown). Eyepiece 240 is disposed on proximal end 230, and can receive light images from distal end 220 via shaft 210. Shaft 210 includes light and/or image transmission components (not shown) as are known in the art.

The components of endoscope 220 are typical and merely descriptive for illustrating applications of endoscope heating device 100. Those having skill in the art will understand that other arrangements are possible without departing from the invention.

Heating element 110 is disposed at distal end 220 of endoscope 200. Electrical conductors 120, 120' are disposed along shaft 210 of endoscope 200 and are connected to power supply 130, which is disposed in the area of proximal end 230. Heating element 110 and/or electrical conductors 120, 120' may be attached to the exterior of endoscope 220 using a suitable adhesive or other attachment (not shown). The adhesive may be biocompatible and/or sterilizable as required by the particular application. Optionally, heating element 110 and/or electrical conductors may be disposed beneath a surface or a covering (not shown) of endoscope 200. The insulation 140 has the shape of a flexible sheet which can be wrapped or posed about the outer surface of the distal end area of the endoscope 200.

In some applications, heating device 100 may be installed on a boroscope or other device incorporating optics at a distal end that are subject to fogging. Other device applications including devices other than endoscopes will be evident to those having skill in the art.

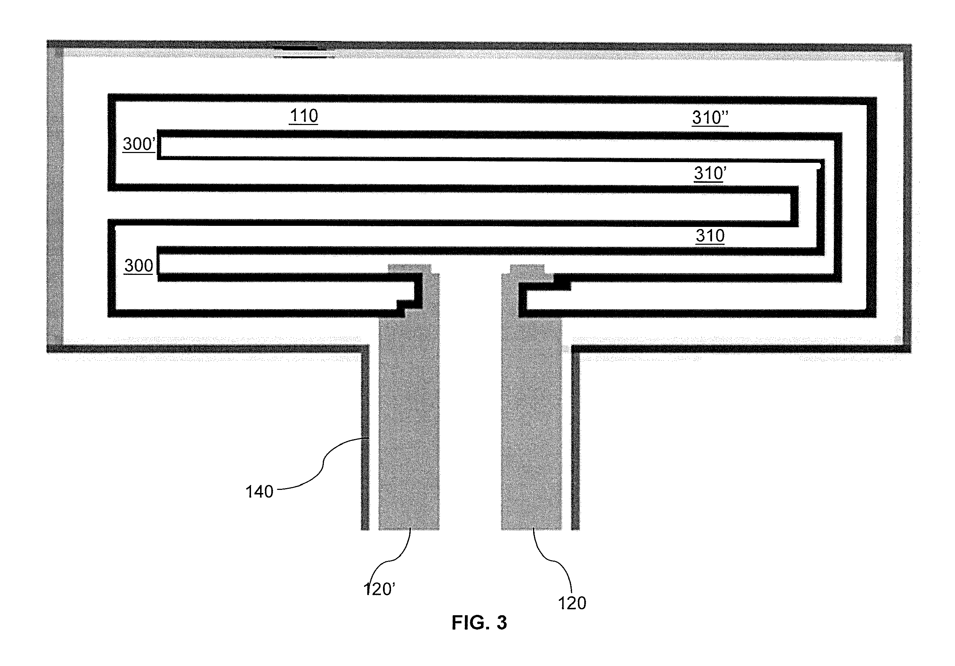

FIG. 3 is an enlarged view of resistive heating element 110 and its surrounding structures, illustrating an example implementation of heating element 110. Here, element 110 is arranged such that it describes a series of bends 300, 300', connected by a series of extending portions, 310, 310' 310". The extending portions 310, 310' 310" are longer than the bends 300, 300'. Element 110 and its surrounding structures are disposed with respect to electrical conductors 120 and 120' so that when installed on an endoscope as shown in FIG. 2, the extending portions 310, 310' 310" extend around a certain arc of the circumference of the distal end of the endoscope shaft. Likewise, the bends 300, 300' are disposed such that they are parallel with, tangent to, or otherwise substantially coincide with the longitudinal axis of the endoscope shaft (not shown).

The implementation of element 110 shown in FIG. 3 can also have the advantage of providing even heating to an optical element disposed in the distal end of an endoscope coaxial with the longitudinal axis, such as an objective lens or window (not shown) by providing lengths of the element 110 which may extend nearly completely around the distal end of the endoscope in the circumferential direction. Uneven heating of the lens may warp the lens due to uneven thermal expansion, resulting in undesirable optical aberrations that can interfere with surgery or other delicate operations.

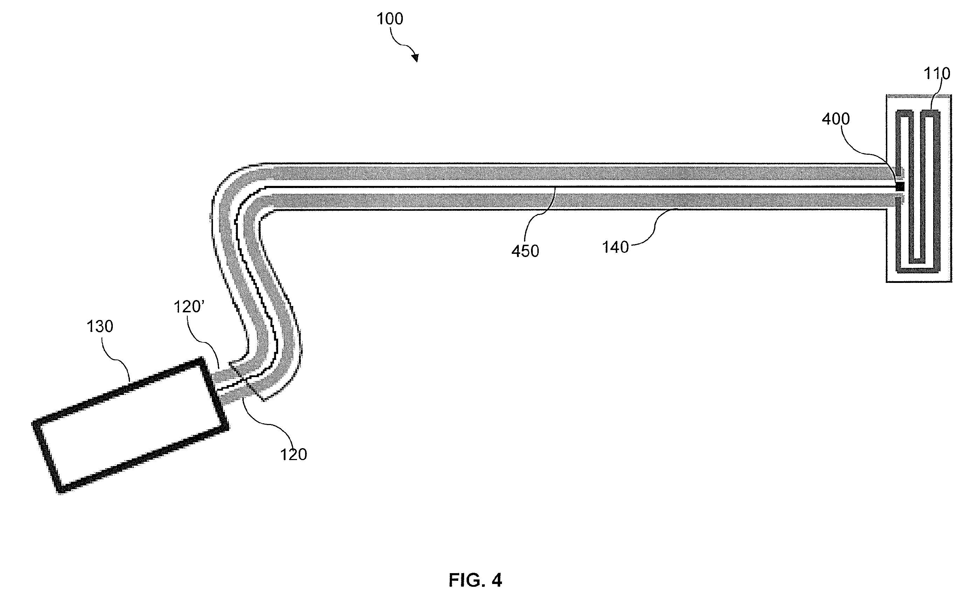

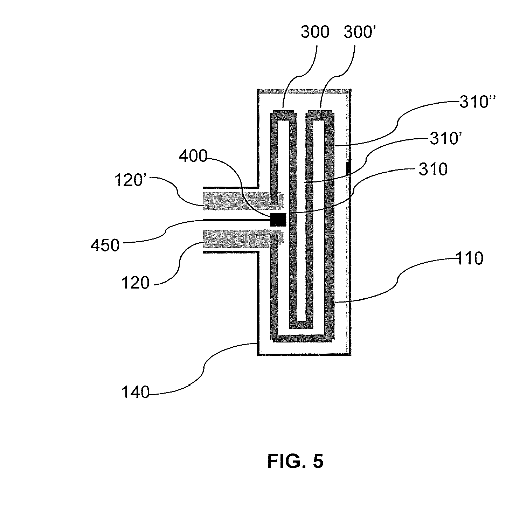

FIGS. 4 and 5 illustrate the endoscope heating device 100 as illustrated in FIGS. 1 - 3, optionally configured to incorporate a temperature sensor 400.

Temperature sensor 400 may include any suitable thermometer, thermocouple, microbolometer, quartz thermometer, resistance temperature detector, thermistor, or other device for detecting the temperature of heating element 110 and/or an endosope or other device to which heating element 110 is installed (not shown).

Temperature sensor 400 may be located on or within the insulation 140 which covers the heating element 110. Optionally, temperature sensor 400 can be installed on an endoscope or other device (not shown) beneath heating element 110, or atop heating element 110. Optionally, temperature sensor 400 can be installed on the distal end of the device. Optionally temperature sensor 400 may be an array of sensors disposed in any of these locations about heating element 110. The use of multiple sensors can have the advantage of providing gradient detection and error correction capabilities as well as fault tolerance.

Temperature sensor lead 450 connects temperature sensor 400 to power source 130. Temperature sensor 400 transmits signals through temperature sensor lead 450 which reflect the temperature at the sensor 400, which in turn reflects the temperature of heating element 110 and any endoscope or other object to which heating element 110 is installed (not shown). In applications incorporating a temperature sensor 400, power source 130 includes appropriate control circuitry to receive and interpret signals from temperature sensor 400. Power source 130 may incorporate a negative feedback servomechanism, thermostat, controller, or other suitable device which can use signals received from temperature sensor 400 to maintain a desired temperature at heating element 110.

In some embodiments, multiple heating elements (not shown) similar to heating element 110 may be provided, and may be disposed in an interleaved or alternating pattern with heating element 110. This can have the advantage of providing added fault tolerance in the event of a failure of one heating element. In addition, in some arrangements, additional heating elements (not shown) can be used to balance temperature gradients which may arise in the endoscope or other device to which the heating elements are attached (not shown) particularly in implementations which incorporate multiple temperature sensors disposed to detect such temperature gradients (not shown). These arrangements can have the advantage of preventing temperature dependent warping of optics that are heated by the system. These arrangements may also have the advantage of preventing or removing partial fogging that may occur in the presence of a temperature gradient that might otherwise be either uncorrectable due to the use of a single heating element or undetectable due to the use of a single temperature sensor.

In some embodiments, heating element 110 may incorporate a temperature safety element such as a thermal fuse element or portion (not shown) or other safety feature which interrupts the heating power if heating element 110 exceeds a certain temperature, for example, a temperature above which tissue may be damaged. Optionally, a temperature safety element (not shown) may be incorporated into the power supply 130.

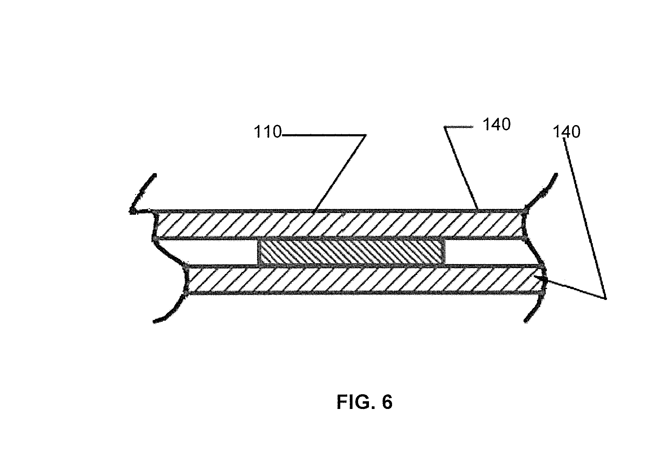

FIG. 6 illustrates a cross-sectional view of heating element 110 as illustrated in FIGS. 1 - 5, shown sandwiched between insulation 140 according to aspects of the invention. Electrical conductors 120, 120' may likewise be sandwiched between insulation 140. Optionally, insulation 140 may completely encase heating element 110 or electrical conductors 120, 120'.

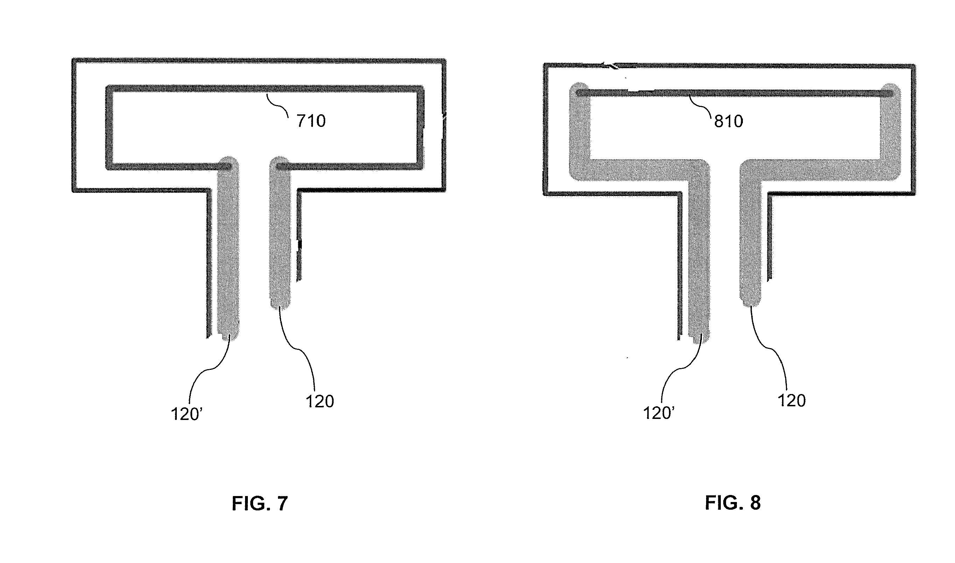

FIG. 7 illustrates heater element 710, which is substantially similar to heater element 110 (FIGS. 1 - 6) except in that heater element 710 represents an alternative geometry for a heater element according to aspects of the invention.

FIG. 8 illustrates heater element 810, which is substantially similar to heater element 110 (FIGS. 1 - 6) except in that heater element 810 represents an alternative geometry for a heater element according to aspects of the invention.

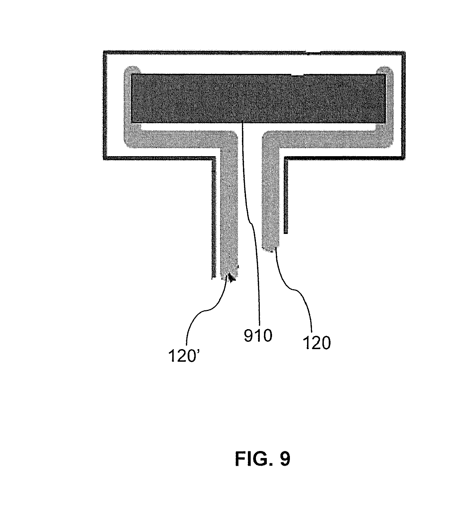

FIG. 9 illustrates heater element 910, which is substantially similar to heater element 110 (FIGS. 1 - 6) except in that heater element 910 represents an alternative geometry for a heater element according to aspects of the invention.

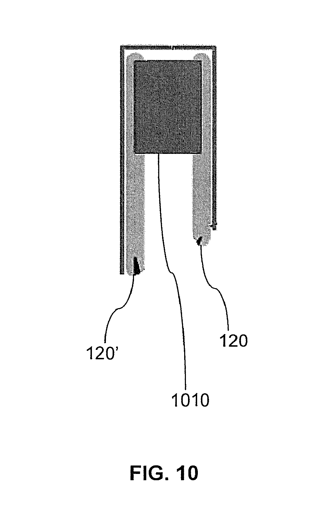

FIG. 10 illustrates heater element 1010, which is substantially similar to heater element 110 (FIGS. 1 - 6) except in that heater element 1010 represents an alternative geometry for a heater element according to aspects of the invention.

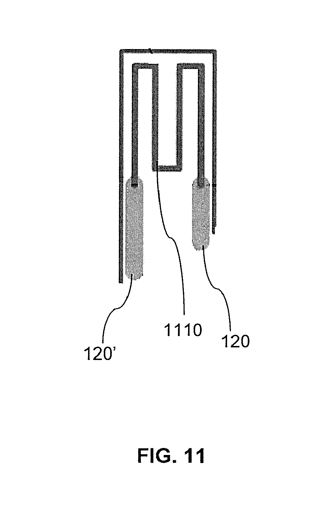

FIG. 11 illustrates heater element 1110, which is substantially similar to heater element 110 (FIGS. 1 - 6) except in that heater element 1110 represents an alternative geometry for a heater element according to aspects of the invention.

Although the invention has been described with reference to a particular arrangement of parts, features and the like, these are not intended to exhaust all possible arrangements or features, and indeed many modifications and variations will be ascertainable to those of skill in the art.

## Claims

1. A system for reducing fogging from an endoscope comprising:
an endoscope having a distal end; and,
a heating module disposed over the distal end of the endoscope on an outside surface of the endoscope, which includes
a heating element that is
positioned between two insulating ribbons.

2. The system of claim 1, wherein the heating element is arranged in a series of elongated portions extending circumferentially around the distal end of the endoscope connected by bend portions that are parallel to a longitudinal axis of the endoscope or tangent to its circumference.

3. The system of claims 1 or 2, wherein the heating element comprises semi-conductive ink.

4. The system of claims 1 or 2, wherein the heating element comprises a semi-conductive film.

5. The system of anyone of claims 1 - 4, wherein the heating element is arranged as a single segment of heating material.

6. The system of anyone of claims 1 - 5, wherein the heating module is removably attached to an outside surface of the endoscope.

7. The system of anyone of claims 1 - 6, wherein the heating module is adhered to an outside surface of the endoscope.

8. The system of anyone of claims 1 - 7, wherein the heating element comprises a material with a resistance having a positive temperature coefficient.

9. The system of anyone of claims 1 - 8, wherein the heating module comprises a biocompatible insulating material.

10. The system of anyone of claims 1 - 9, wherein the heating element is self-limiting such that its temperature will not exceed a certain maximum.
